# EUROPEAN PATENT APPLICATION

(11) **EP 1 505 138 A2**
(43) Date of publication of application: **09.02.2005**
(21) Application number: 04018445.9
(22) Date of filing: 04.08.2004
(51) Int. Cl.: C09J 9/00, C09J 11/00

(54) **Hot melt adhesive containing a neutralizing additive**

(30) Priority: 06.08.2003 US 635139
(71) Applicant: National Starch and Chemical Investment Holding Corporation, New Castle, Delaware 19720 (US)
(72) Inventor: Harwell, Michael G., Hillsborough New Jersey 08844 (US); Thatcher, Jennifer, North Brunswick New Jersey 08902 (US); McCauley, Robert, Independence Kentucky 41051 (US)
(74) Representative: Hagemann, Heinrich, Dr. rer. nat., Dipl.-Chem.

(57) **Abstract**

Hot melt adhesives comprising an effective amount of a neutralizing additive is useful in the manufacture of absorbent articles, such as diapers/training pants and feminine hygiene products. The added material has a closed cup flash point of greater than 100°F, most preferably a closed cup flash point of greater than 200°F.

## Description

### FIELD OF THE INVENTION

The present invention relates to odor neutralized adhesives. In particular, the invention relates to hot melt adhesives comprising neutralizers, to the use of a neutral odor hot melt adhesive to prepare a disposable absorbent product and a disposable absorbent articles comprising neutral odor hot melt adhesives.

### BACKGROUND OF THE INVENTION

Disposable absorbent products currently find widespread use in many applications. For example, diapers, training pants, incontinent garments, feminine hygiene pads, i.e., catamenial pads, sanitary napkins, panty liners, panty shields and the like, underarm shields and health care products such as surgical drapes or wound dressings. Such articles are designed to absorb body fluids, such as urine, menses, blood, perspiration and other excrements discharged by a body.

A typical disposable absorbent product generally comprises a composite structure including a fluid-permeable topsheet, a fluid absorbent core and a fluid-impermeable backsheet. These disposable absorbent products, when appropriate, usually include some type of fastening system for fitting the product onto the wearer.

Adhesives, often referred to as construction adhesives, are generally used to join the different parts of the disposable absorbent product together. Hot melt adhesives are preferably used for this purpose since such adhesives allow for cost and time efficient manufacturing since there is no evaporation step necessary as is the case for water-based or solvent-based adhesive systems.

Hot melt adhesives, however, are known to exhibit malodors due to the source and composition of the raw materials used to make them. Several processing steps have been commonly used in the refining of these raw materials to reduce their base odor. Separation techniques such as distillation to separate highly volatile materials or reaction techniques such as hydrogenation to chemically change high odor species. Both approaches are somewhat effective, but are costly and in practice can only reduce the malodor of the raw materials to a certain extent.

It is known that some fragrances or perfumes are also compatible with hot melt adhesives. These materials can be used to mask the base odor of the hot melt adhesive by adding a perfume odor on top of the base odor of the adhesive. Typically, the odor of the hot melt is extremely sensitive to the type and level of perfume added and commonly the final odor of the adhesive is that of the perfume.

There continues to be a need in the art for effective ways to reduce or eliminate the base odor of hot melt adhesives, in particular methods that do not use perfumes or fragrances to mask the odor of the base adhesive. The current invention addresses this need in the art.

### SUMMARY OF THE INVENTION

A class of additives that act as odor neutralizers for hot melt adhesives has been discovered. The invention provides neutral odor compositions and a process for the effective neutralization of hot melt adhesives odor.

The invention provides hot melt adhesives having contained therein molecules of at least one neutralizing material.

One aspect of the invention is directed to a hot melt adhesive comprising a neutralizing material. Most preferably the neutralizing material has a closed cup flash point of greater than 100°F. In one embodiment of the invention, the hot melt adhesive comprises Quest material Q-29071. In another embodiment of the invention, the hot melt adhesive comprises Quest material Q-29072.

Another aspect of the invention is directed to a method of reducing or counteracting malodors, in particular malodors present in raw materials used in the manufacture of hot melt adhesives (e.g., tackifier component), malodors present in formulated hot melt adhesives, and the like. The method comprises added to a composition in need thereof an effective amount of a neutralizing material effected to reduce or counteract the odor to a desired degree, preferably at least about 50 % counteraction or reduction, more preferably at least about 80% counteraction or reduction, even more preferably about 100% counteraction or reduction. In a preferred method, the neutralizing material is added to a hot melt adhesive or a component thereof (e.g., tackifier and/or mineral oil). The degree of desired reduction or counteraction will depend on the desired end use and/or quality of the raw materials (e.g., tackifiers) used in the manufacture of the hot melt adhesive.

Still another aspect of the invention is directed to an article of manufacture comprising a neutralizing material. In one embodiment, the article is a disposable absorbent article of manufacture comprising a liquid-permeable topsheet, a liquid-impermeable backsheet, a fluid-absorbent core material positioned between the topsheet and the backsheet and a hot melt adhesive containing a neutralizing material. A particularly preferred aspect of the invention is directed to disposable absorbent garments such as, for example, diapers for use in infants and toddlers, adult incontinent pads and feminine hygiene products.

Yet another aspect of the invention is directed to a process for bonding one substrate to a second substrate, wherein the first and second substrate may be the same or different. In one embodiment, a tissue or a nonwoven substrate is bonded to similar or dissimilar substrates in the construction of a disposable absorbent product. The process comprises applying to at least one substrate a molten hot melt adhesive composition and bonding the substrate together, wherein the hot melt adhesive comprises a neutralizing material.

### DETAILED DESCRIPTION OF THE INVENTION

The invention provides hot melt adhesives comprising neutralizing compositions, processes for effectively neutralizing malodors present in formulated hot melt adhesives, and articles of manufacture comprising neutralized hot melt adhesives.

The neutralizing compositions, also referred to herein as neutralizing additives or neutralizing materials, may be added to hot melt adhesives without a requirement for encapsulation or other protective means, and are capable of remaining stable in the adhesives prior to and after use without significant increase in adhesive odor. The neutralizing composition is used in the process at a suitable level depending on the product to achieve the desired effect in terms of malodor reduction properties of the product.

The term "malodor" is used herein to refer to smells or odors generally regarded as undesirable or unpleasant in nature. Neutralized hot melts have utility in a variety of applications where reduced, low or no odor is required. The adhesives of the invention may be used, for example, in bookbinding, packaging and converting, labeling, and are especially useful in the area of disposable articles such as diapers, feminine pads, adult diapers and the like.

A perfume or fragrancing composition is generally recognized in the art as possessing odors in its own right, and when used as a malodor reduction agent functions to cover-up or mask the malodor with a stronger, more pleasant scent. In contrast, the addition of the neutralizing material of the invention functions to lower malodor of the base adhesive without imparting a significant amount of fragrance. That is, the malodor is counteracted rather than masked.

The subject invention is concerned particularly with reduction of malodor (i.e., reducing the perceived intensity of a malodor) not by masking the malodor (e.g., dominating the malodor with a stronger odor) but by counteracting or neutralizing the malodor in a way that reduces perceived malodor intensity without the need for an intense perfume, or a perfume with a pronounced idiosyncratic odor character such as eucalyptus or wintergreen.

In one embodiment of the invention, a disposable absorbent product is provided, which disposable absorbent product comprises (1) a liquid-permeable topsheet, (2) a liquid-impermeable backsheet, which topsheet may be attached to the backsheet, (3) an absorbent structure positioned between the topsheet and the backsheet, and (4) an odor-neutralized adhesive.

The absorbent structure will typically comprise a nonwoven fabric. A nonwoven fabric is defined as an interlocking fiber network characterized by flexibility, porosity and integrity. The individual fibers used to compose the nonwoven fabric may be synthetic, naturally occurring, or a combination of the two. The individual fibers may be mechanically, chemically, or thermally bonded to each other. Nonwovens are used commercially for a variety of applications including insulation, packaging, household wipes, surgical drapes, medical dressings, and in disposable articles such as diapers, adult incontinent products and sanitary napkins. Tissue is a closely related material in which the individual fibers may or may not be chemically bonded to one another.

The adhesives according to the invention may be formulated for use as, e.g., positioning adhesives, core adhesives or elastic adhesives, and are particularly suitable for use in the manufacture or articles, including but not limited to disposable absorbent products, such as diapers, adult incontinent products, bed pads; sanitary napkins, and in other absorbent products, such as, bibs, wound dressings, and surgical capes or drapes, which are used to absorb a liquid, such as water and saline, and body liquids, such as urine, menses, and blood. The odor-neutralized adhesive of the invention may be used to adhere the nonwoven or tissue to another substrate or component. The second substrate may be another nonwoven, tissue, or an unrelated material.

The adhesive may be used to attach the topsheet to the backsheet. Alternatively, the adhesive may be used to adhere either the topsheet or the backsheet to other components of the disposable absorbent product, such as tissue layers, leg flaps, fastening ears, tapes, or tabs, or other components typically used to construct a disposable absorbent product that are well known to one skilled in the art.

Those skilled in the art will recognize materials suitable for use as the topsheet and backsheet.

Exemplary of materials suitable for use as the topsheet are liquid-permeable materials, such as spunbonded polypropylene or polyethylene having a basis weight of from about 15 to about 25 grams per square meter.

Backsheets often used in disposable absorbent products are generally prepared from liquid-impermeable materials which function to contain liquids, such as water, urine, menses, or blood, within the absorbent core of the disposable absorbent product and to protect bedding and/or a wears' outer garments from soiling. Materials useful as a backsheet in a disposable absorbent product are generally impermeable to liquid but are permeable to vapor. Examples are liquid-impervious materials such as polyolefin films, e.g., polypropylene and polyethylene, as well as vapor-pervious materials, such as microporous polyolefin films, sometimes referred to as breathable films.

A particularly desirable backsheet material is a film comprising a polyolefin polymer such as a linear low density polyethylene and a filler. As used herein a "filler" is meant to include particulates and other forms of materials which can be added to the film polymer extrusion blend and which will not chemically interfere with or adversely affect the extruded film but which are able to be uniformly dispersed throughout the film. When the film is stretched during processing, the filler generally causes a network of holes to be formed in the film. Such holes are generally small enough to prevent the passage of a liquid, but are generally large enough to allow vapor to pass through the holes. Generally the fillers will be in particulate form and usually will have somewhat of a spherical shape with average particle sizes in the range of about 0.1 to about 7 microns. Both organic and inorganic fillers may be used in the practice of the invention. Examples of fillers include calcium carbonate (CaCO₃), various kinds of clay, silica (SiO₂), alumina, barium sulfate, sodium carbonate, talc, magnesium sulfate, titanium dioxide, zeolites, aluminum sulfate, cellulose-type powders, diatomaceous earth, magnesium sulfate, magnesium carbonate, barium carbonate, kaolin, mica, carbon, calcium oxide, magnesium oxide, aluminum hydroxide, pulp powder, wood powder, cellulose derivatives, chitin and chitin derivatives.

Odor-neutralizing compositions which may be incorporated into hot melt adhesives in accordance with the invention are materials that have a closed cup flash point greater than about 100°F, preferably greater than about 150°F, more preferably greater than about 200°F as determined by ASTM method D93-00 (Flash Point by Pensky-Martens Closed Cup Tester). Any neutralizing material having a flashpoint greater than 100°F may be used in the practice of the invention. Such neutralizers are commercially available. An example of a neutralizer that may be used is commercially available from Quest (Q-29071 and Q-29072). Both of these materials have closed cup flash point of greater than 200°F and function to effectively neutralized the odor present in a hot melt adhesive.

The odor-neutralized composition(s) are added to the hot melt adhesive composition without the requirement for encapsulation or other protective means. The term nonencapsulated is used as conventional in the art and means that the neutralizing material or composition is not enclosed in a protective cover or shell. Thus, the odor-neutralized compositions can be formulated into the hot melt adhesive without the prior encapsulation thereof.

The neutralizing compositions may be added to virtually any hot melt type adhesive. Particularly preferred for use in the practice of the invention are low application temperature hot melt adhesive formulations, i.e., formulations that can be applied at temperatures below about 300°F, more preferably at about 250°F and down to about 200°F. Low application temperature hot melt adhesives are commercially available from National Starch and Chemical Company, Bridgewater, NJ.

Any base polymer suitable for use in formulating hot melt adhesives, as are well known to those skilled in the art may be used in the practice of the invention. Such polymers include amorphous polyolefins, ethylene-containing polymers and rubbery block copolymers, as well as blends thereof. Hot melt adhesive compositions based on ethylene/vinyl acetate copolymers, isotactic or atactic polypropylene, styrene-butadiene, styrene-isoprene, or styrene-ethylene-butylene A-B-A or A-B-A-B block copolymers or mixtures thereof may be used. In addition to the base polymer, the hot melt adhesive compositions of the invention may also contain tackifiers, oils and/or waxes as well as conventional additives including stabilizers, anti-oxidants, pigments and the like.

In more detail, the neutralizing compositions may be added to adhesives based on rubbery block copolymers. These polymers include the block or multi-block copolymers having the general configuration: A-B-A or A-B-A-B-A-B- wherein the polymer blocks A are non-elastomeric polymer blocks which, as homopolymers have glass transition temperatures above 20°C, while the elastomeric polymer blocks B are butadiene or isoprene or butadiene isoprene which is partially or substantially hydrogenated. Further, they may be linear or branched. Typical branched structures contain an elastomeric portion with at least three branches which can radiate out from a central hub or can be otherwise coupled together.

The non-elastomeric blocks may comprise homopolymers or copolymers of vinyl monomers such as vinyl arenes, vinyl pyridines, vinyl halides and vinyl carboxylates, as well as acrylic monomers such as acrylonitrile, methacrylonitrile, esters of acrylic acids, etc. Monovinyl aromatic hydrocarbons include particularly those of the benzene series such as styrene, vinyl toluene, vinyl xylene, ethyl vinyl benzene as well as dicyclic monovinyl compounds such as vinyl naphthalene and the like. Other non-elastomeric polymer blocks may be derived from alpha olefins, alkylene oxides, acetals, urethanes, etc.

The elastomeric block component of the copolymer may be isoprene or butadiene which may or may not be hydrogenated. This hydrogenation may be either partial or substantially complete. Selected conditions may be employed for example to hydrogenate the elastomeric block while not so modifying the vinyl arene polymer blocks. Other conditions may be chosen to hydrogenate substantially uniformly along the polymer chain, both the elastomeric and non-elastomeric blocks thereof being hydrogenated to practically the same extent, which may be either partial or substantially complete.

Typical of the rubbery block copolymers useful herein are the polystyrene-polybutadiene-polystyrene,polystyrene-polyisoprene-polystyrene and e.g., polystyrene-poly-(ethylenebutylene)-polystyrene and polystyrene-poly-(ethylenepropylene)-polystyrene. These copolymers may be prepared using methods taught, for example, in U.S. Patent Nos. 3,239,478; 3,427,269; 3,700,633; 3,753,936; and 3,932,327. Alternatively, they may be obtained from Kraton Polymers under the trademarks Kraton 1101, 1102, 1107, 1650, 1652 and 1657; from Polimeri Europa under the Europrene Sol-T tradenames; and from Firestone under the tradename Stereon 840A.

Ethylene containing polymers are also commonly used for disposable applications and can be neutralized by the addition thereto of the neutralizing compositions in accordance with the teachings of the invention. The adhesive of the invention may comprise at least one ethylene copolymer, and may comprise a blend of two or more polymers. The term ethylene copolymer, as used herein, refers to homopolymers, copolymers and terpolymers of ethylene. Examples of ethylene copolymers include copolymers with one or more polar monomers which can copolymerize with ethylene, such as vinyl acetate or other vinyl esters of monocarboxylic acids, or acrylic or methacrylic acid or their esters with methanol, ethanol or other alcohols. Included are ethylene vinyl acetate, ethylene methyl acrylate, ethylene n-butyl acrylate, ethylene acrylic acid, ethylene methacrylate and mixtures and blends thereof. Random and block copolymers, as well as blends thereof may be used in the practice of the invention.

Other adhesive compositions may be prepared according to the invention using, as a base polymer, amorphous polyolefins or blends thereof. Amorphous polyolefins are made by the stereospecific polymerization of polypropylene. Suitable commercial products include Eastman's P 1010. Copolymers of amorphous polypropylene and ethylene, amorphous polypropylene and butene and amorphous polypropylene and hexene are suitable as a base polymer, as are terpolymers of propylene, butene and ethylene. Commercial examples include Rextac 2315 (copolymer of amorphous polypropylene and ethylene) available from Rexene, Rextac 2730 (copolymer of amorphous polypropylene and butene) also available from Rexene and Vestoplast 750 and 708 (terpolymers of amorphous propylene, butene and ethylene) available from Huls.

Blends of any of the above base materials, such as blends of ethylene n-butyl acrylate and ethylene vinyl acetate and ethylene vinyl acetate and atactic polypropylene may also be used to prepare hot melt adhesive compositions. In all cases, the adhesives may be formulated with tackifying resins, plasticizers, waxes and/or other conventional additives in varying amounts as are known to those skilled in the art and as required for particular formulations, e.g., a pressure sensitive adhesive formulation.

Tackifying resins useful in the adhesive compositions of this invention include hydrocarbon resins, synthetic polyterpenes, rosin esters, natural terpenes, and the like. More particularly, and depending upon the particular base polymer, the useful tackifying resins may include any compatible resins or mixtures thereof such as natural and modified rosins including, for example, as gum rosin, wood rosin, tall oil rosin, distilled rosin, hydrogenated rosin, dimerized rosin, and polymerized rosin; glycerol and pentaerythritol esters of natural and modified rosins, including, for example as the glycerol ester of pale, wood rosin, the glycerol ester of hydrogenated rosin, the glycerol ester of polymerized rosin, the pentaerythritol ester of hydrogenated rosin, and the phenolic-modified pentaerythritol ester of rosin; copolymers and terpolymers of natured terpenes, including, for example, styrene/terpene and alpha methyl styrene/terpene; polyterpene resins having a softening point, as determined by ASTM method E28-58T, of from about 80°C to 150°C; phenolic modified terpene resins and hydrogenated derivatives thereof including, for example, the resin product resulting from the condensation, in an acidic medium, of a bicyclic terpene and a phenol; aliphatic petroleum hydrocarbon resins having a Ball and Ring softening point of from about 70°C to 135°C; aromatic petroleum hydrocarbon resins and the hydrogenated derivatives thereof; and alicyclic petroleum hydrocarbon resins and the hydrogenated derivatives thereof. Mixtures of two or more of the above described tackifying resins may be required for some formulations.

Various plasticizing or extending oils are also present in the composition in amounts of 5% to about 30%, preferably 5 to 25%, by weight in order to provide wetting action and/or viscosity control. Even higher levels may be used in cases where block copolymer containing hydrogenated mid-block are employed as the adhesive base polymer. The above broadly includes not only the usual plasticizing oils but also contemplates the use of olefin oligomers and low molecular weight polymers as well as vegetable and animal oil and their derivatives. The petroleum derived oils which may be employed are relatively high boiling materials containing only a minor proportion of aromatic hydrocarbons (preferably less than 30% and, more particularly, less than 15% by weight of the oil). Alternatively, the oil may be totally nonaromatic. The oligomers may be polypropylenes, polybutenes, hydrogenated polyisoprene, hydrogenated polybutadiene, or the like having average molecular weights between about 350 and about 10,000. Vegetable and animal oils include glyceryl esters of the usual fatty acids and polymerization products thereof.

Various petroleum derived waxes may also be used in amounts less than about 15% by weight of the composition in order to impart fluidity in the molten condition of the adhesive and flexibility to the set adhesive, and to serve as a wetting agent for bonding cellulosic fibers. The term "petroleum derived wax" includes both paraffin and microcrystalline waxes having melting points within the range of 130°F to 225°F as well as synthetic waxes such as low molecular weight polyethylene or Fisher-Tropsch waxes.

An antioxidant or stabilizer may also be included in the adhesive compositions described herein in amounts of up to about 3% by weight. Among the applicable antioxidants or stabilizers are high molecular weight hindered phenols and multifunctional phenols such as sulfur and phosphorous-containing phenols. Representative hindered phenols include: 1,3,5-trimethyl 2,4,6-tris (3,5-di-tert-butyl-4-hydroxy-benzyl)benzene; pentaerythritol tetrakis-3(3,5-di-tert-butyl-4-hydroxyphenyl)-propionate; n-octadecyl-3,5-di-tert-butyl-4-hydroxyphenol)-propionate; 4,4'-methylenebis (2,6-tert-butylphenol); 4,4'-thiobis (6-tert-butyl-o-cresol); 2,6-ditertbutylphenol; 6-(4-hydroxyphenoxy)-2,4-bis(n-octyl-thio)-1,3,5-triazine; di-n-octadecyl 3,5-di-tert-butyl-4-hydroxy-benzyl-phosphonate; 2n-octylthio)-ethyl 3,5-di-tert-butyl-4hydroxybenzoate and sorbitol hexa[3-(3,5-ditert-butyl-4-hydroxyphenyl)-propionate].

Other additives conventionally used in hot melt adhesives to satisfy different properties and meet specific application requirements also may be added to the adhesive composition of this invention. Such additives include, for example, fillers, pigments, flow modifiers, dyestuffs, which may be incorporated in minor or larger amounts into the adhesive formulation, depending on the purpose.

Hot melt adhesives may be prepared using techniques known in the art. Typically, the adhesive compositions are prepared by blending the components in the melt at a temperature of about 100° to 200°C until a homogeneous blend is obtained, approximately two hours. Various methods of blending are known and any method that produces a homogeneous blend is satisfactory.

The neutralizer composition may be added, with stirring, during the preparation of the hot melt adhesive. If desired, the neutralizer may be added to the tackifier component (e.g., during the manufacture thereof), or may conveniently be added to the mineral oil additive, used in preparing the hot melt to be neutralized. The neutralizer is added in an amount effective to neutralize the malodor common with many hot melt adhesives to the user of the adhesive following cooling and to the user of any end product manufactured using the neutralized adhesive of the invention. Typically, the neutralizer will be present in an amount of about 0.001 to about 1.0 parts, preferably about 0.02 to about 0.05 parts, per 100 parts of the adhesive composition. The amount of neutralizer added will typically depend on the amount of neutralization desired or required for a particular application or end use.

The resulting adhesives of the present invention are characterized by their ability to provide a durable bond to a nonwoven or tissue article and otherwise meet the unique requirements of the application, such flexibility, non-staining, and machinable viscosity, and by their reduced or odor-free properties

The adhesive is applied to a substrate while in its molten state and cooled to harden the adhesive layer. The adhesive product can be applied to a substrate such as a nonwoven article by a variety of methods including coating or spraying in an amount sufficient to cause the article to adhere to another substrate such as tissue, nonwoven, or an unrelated material such as a low density polyolefin or other conventionally employed substrates.

### EXAMPLES

The following examples illustrate the production of suitable odor-neutralized hot melt adhesives. In the examples, all parts are by weight and all temperatures in degree Celsius unless otherwise noted. Test procedures used herein are as follows:

### TEST PROCEDURES

### Viscosity

The viscosity as used herein is a Brookfield viscosity measured using a Brookfield viscometer model No. DV-II with spindle no. 27 at 20 rpm.

### Tensile Strength

Tensile Strength (Ultimate & Yield) was determined using dumbbell hot melt molds and an instron Unit. In this test hot melt was poured molten into the mold, allowed to cool to room temperature for 24 hours and pulled in an Instron unit at 12 inches/minute.

### Mettler Ring & Ball Softening Point

This test method describes the method used to determine the softening point of a thermoplastic composition in the range 50-150°C using a Mettler Ring & Ball Assembly (model # FP90).

### Peel Strength

Peel strength was measured using T-peel bonds on an Instron unit at 12"/min. Bonds were made between polyethylene film and polypropylene nonwoven using a high speed laminator.

### Odor

Odor was determined using a comparison test between a control sample with no neutralizer and a neutralized sample. The odor was described by its intensity and its character. Intensity is defined as the overall strength of the smell (e.g., strong, moderate, weak or slight, etc.). Character is defined as the perceived description of the of the smell (e.g., clean, lavender, no scent, etc.).

Reduction in malodor was measured experimentally by assessing these test materials for their effectiveness in reducing (counteracting) malodors in small scale headspace assessments carried out by trained sensory assessors. The sensory panel consists of a panel of 3-5 individuals who are trained to identify individual odor characters in complex mixtures, and to rate their perceived intensity. The level of efficiency of the panel is continuously monitored to ensure a high level of accuracy and reproducibility.

During testing all variables other than those actually under test are controlled as carefully as possible. Samples are prepared so that they are, as far as possible, identical apart from their differences in odor. When presented to panelists they are presented in random order. A minimum of 5 assessments were collated for each sample.

Hot melt adhesives containing a malodor reducing ingredient (e.g. fragrance, neutralizing agent) were placed a 4 ounce glass vessel. The vessel is closed and allowed to equilibrate for half an hour before assessment.

Each panel member assessed each sample for the intensity and character of any smell that can be perceived in the headspace of the glass vessel. Hidden blanks (malodor but no perfume or other agent) were included as internal controls. The results of each of the panelists were normalized and averaged to give a consensus rating across the whole panel.

### Example 1

A formulated odor-neutralized adhesive was prepared by adding 0.02% Quest neutralizer Q-29072, with stirring at 130°C, to 100% hot melt adhesive 34-520A, a hot melt adhesive commercially available from National Starch and Chemical Company. 34-520A is a rubber-based hot melt adhesive containing a hydrocarbon tackifier and oil, and is used in the construction of disposable articles.

### Example 2

A formulated odor-neutralized adhesive was prepared by adding 0.05% Quest neutralizer Q-29071 to 100 % of hot melt adhesive 34-184A with stirring at 150°C. 34-184A is a rubber-based hot melt adhesive containing a hydrocarbon tackifier and an oil, that is available from National Starch and Chemical Company and used for the construction of disposable articles. Viscosity, color, tensile strength (ultimate and yield) and Ring & Ball tests were carried out on the 34-184A adhesive composition and the neutralized 34-184A adhesive composition. Results are shown in Table 1.

Thereafter, the adhesives were placed in covered glass jars in an oven at 150°C for approximately 72 hours. The samples were then removed from the oven and allowed to cool to ambient conditions. The samples were covered overnight and then evaluated for odor. A description of the intensity and character of each sample was recorded and is shown in Table 1.

**Table 1**

| | | 34-184A | 34-184A+Q71 |
|---|---|---|---|
| Viscosity @ 160°C (cps) | | 3170 | 3060 |
| Tensile yield (psi) | | 14 | 16 |
| Tensile strength (psi) | | 118 | 119 |
| Softening point (°C) | | 91.2 | 90.7 |
| Initial bond strength T-peel avg, (g/sq. in.) | | 93 | 91 |
| Odor results: | | | |
| | • intensity | | |
| initial | | moderate | very weak |
| aged 24 hours | | moderate | very weak |
| aged 48 hours | | strong | weak |
| aged 72 hours | | very strong | weak |
| | • character | | |
| initial | | resin/oil | neutral |
| aged 24 hours | | resin/oil | neutral |
| aged 48 hours | | resin/oil | neutral |
| aged 72 hours | | resin/oil | neutral |

From this example it is seen that the malodor typical of hot melt adhesives can be effectively neutralized without affecting the properties of the hot melt adhesive.

### Example 3

Neutralizers Q-71 and Q-72 and a fragrance (named Tender), also available from Quest International, were added to the adhesive 34-520A at 0.02%. Cooled samples were smelled after initial preparation and after 24 hours at 130°C in a closed glass jar in an oven. Aged odor results are shown in Table 2.

**Table 2**

| | Initial | | Aged | |
|---|---|---|---|---|
| | intensity | character | intensity | character |
| 34-520A | moderate | resin/oil | strong | resin/oil |
| 34-520A+Q-71 | weak | neutral | weak | neutral |
| 34-520A+Q-72 | very weak | neutral | very weak | neutral |
| 34-520A+Tender | strong | floral | strong | floral |

## Claims

1. A hot melt adhesive comprising a neutralizing material.

2. The adhesive of claim 1 wherein the adhesive is a rubber based adhesive containing a hydrocarbon tackifier and/or a terpene tackifier.

3. The adhesive of claim 1 which is a pressure sensitive adhesive.

4. The adhesive of claim 1 which is a low application temperature hot melt adhesive.

5. An article of manufacture comprising the adhesive of claim 1.

6. The article of claim 5 which is a disposable absorbent article comprising a liquid-permeable topsheet, a liquid-impermeable backsheet, a fluid-absorbent core material positioned between the topsheet and the backsheet and a hot melt adhesive containing a nonencapsulated neutralizing material.

7. The article of claim 5 wherein the adhesive is a rubber based adhesive containing a hydrocarbon tackifier and/or a terpene tackifier.

8. The article of claim 6 which is a diaper, a feminine hygiene product or adult incontinent product.

9. A method of reducing or counteracting malodors comprising adding to a malodorous composition an amount of a neutralizing material effective to reduce or counteract the malodor to a desired degree.

10. The method of claim 9 wherein the malodorous composition is a hot melt adhesive.
